# EUROPEAN PATENT APPLICATION

(11) **EP 2 133 088 A2**
(43) Date of publication of application: **16.12.2009**
(21) Application number: 08157868.4
(22) Date of filing: 09.06.2008
(51) Int. Cl.: A61K 36/48, A61P 29/00

(54) **Rooibos and inflammation**

(71) Applicant: Nestec S.A., 1800 Vevey (CH)
(72) Inventor: Philippe, David, 1003, LAUSANNE (CH); Blum-Sperisen, Stéphanie, 1009, PULLY (CH); Roger, Olivier Yves, 1410, CORREVON (CH); Crespy, Vanessa, 1800, VEVEY (CH); Offord Cavin, Elizabeth, 1820, MONTREUX (CH); Ameye, Laurent, 1000, LAUSANNE 26 (CH); Marin-Kuan, Maricel, 1012 Lausanne (CH)
(74) Representative: Marquardt, Ulf

(57) **Abstract**

The present invention relates to the field of inflammatory disorders. In particular the present invention relates to rooibos and rooibos extracts as well as to their use to produce health benefits.

One embodiment of the present invention relates to the use of a composition comprising *Aspalathus linearis* or an extract thereof for the preparation of a product to treat and/or prevent inflammatory disorders.

## Description

The present invention relates to the field of inflammatory disorders. In particular the present invention relates to rooibos and rooibos extracts as well as to their use to produce health benefits.

Inflammation is recognized as a type of nonspecific immune response and is a basic way in which the body reacts to infection, irritation or other injury. Typical symptoms of inflammations are redness, warmth, swelling and pain. Usually, inflammation is tightly regulated by the body. However, if the control pathways of inflammation fail, and inflammation runs unchecked, it can lead to several diseases, such as hay fever, atherosclerosis, and rheumatoid arthritis. Further examples of inflammatory disorders include asthma, autoimmune diseases, chronic inflammation, chronic prostatitis, glomerulonephritis, hypersensitivities, inflammatory bowel diseases, pelvic inflammatory disease, reperfusion injury, osteoarthritis, or vasculitis.

From hay fever alone, which is also known as seasonal allergic rhinitis, about 20% of the people in the UK are affected. Treating and preventing inflammatory disorders is therefore an important object in society today.

Inflammatory disorders are presently generally treated by medication, such as antihistamines, leukotriene receptor antagonists, corticosteroids or cromolyn sodium, all of which may have undesired side effects.

Unwanted inflammatory processes can further be prevented by healthy lifestyle habits such as exercising regularly, not smoking, maintaining a healthy weight, and minimizing stress.

A key factor for preventing inflammatory disorders, however, is the food and drink that is consumed every day.

Consequently, there is a need in the art for a natural substance that can be used to treat or prevent inflammatory disorders.

It was hence the object of the present invention to provide the art with a naturally occurring composition, that is safe to administer and can be consumed every day without undesired side effects, that is available to everybody without having to consult a medical practitioner and that can successfully be used to treat or prevent inflammatory disorders.

The present inventors were surprised to see that they could achieve this object by a use in accordance with claim 1.

Rooibos, Afrikaans for "red bush"; is a member of the legume family of plants and may be used for example to make tea. The scientific name of rooibos is *Aspalathus linearis.* Rooibos tea is often called South African red tea or simply red tea or bush tea. Rooibos tea has been popular in South Africa for generations, but is recently consumed increasingly in countries all over the world.

Rooibos tea is also becoming more and more popular in particular in Western countries among health-conscious consumers, due to its high level of antioxidants.

The present inventors have now found that *Aspalathus linearis* also possesses potent anti-inflammatory properties.

Consequently, one embodiment of the present invention is a composition comprising *Aspalathus linearis* or an extract thereof to treat or prevent inflammatory disorders.

The present invention also relates to the use of a composition comprising *Aspalathus linearis* or an extract thereof for the preparation of a product to treat and/or prevent inflammatory disorders.

The inflammatory disorder that can be treated or prevented according to the present invention can for example be selected from the group consisting of acute inflammations such as sepsis, infections, burns and chronic inflammations such as inflammatory bowel disease, Crohn's disease, ulcerative colitis, necrotizing enterocolitis, asthma, autoimmune diseases, inflammatory bowel syndrome, liver inflammation, alcoholic cirrhosis, allergy, atopy, bone inflammation, arthritis, in particular rheumatoid arthritis and osteoarthritis, systemic lupus, Gougerot-Sjogren's syndrome, Reiter's syndrome, poliomyelitis, dermato-myositis, thyroïditis, Basedow, Hashimoto, type I diabetes, Addison's disease, auto-immunes hepatitis, celiac disease, Biermer's disease, multiple sclerosis, myasthenia, encephalomyelitis, eye inflammation, obesity-associated inflammation, age-related low-grade inflammation, Blau's syndrome, Alzheimer's disease, cardiovascular diseases, atherosclerosis, metabolic syndrome, gingivitis, paronditis, or the symptoms thereof.

In a particular preferred embodiment of the present invention, the composition comprising *Aspalathus linearis* or an extract thereof is used to treat and/or prevent inflammatory disorders of the gut and/or of the cartilage.

For example, the composition comprising *Aspalathus linearis* or an extract thereof may be used to modulate the ratio of cartilage anabolism and cartilage catabolism, in particular to increase the ratio of cartilage anabolism and cartilage catabolism. It may also be used to inhibit cartilage catabolism.

Suitable extracts of *Aspalathus linearis* may be prepared by any means that are known in the art, e.g., by steam extraction, solvent extraction, distillation, pressing or grinding.

It is however preferred if the extract is obtainable, in particular obtained, by extraction with a solvent from *Aspalathus linearis* plant material, in particular by a water extraction or an alcohol/water extraction, for example by a ethanol/water extraction.

The use of water as extraction medium has the advantage that the obtained product can be directly incorporated into final products without having to eliminate the extraction medium first.

The *Aspalathus linearis* extracts may also be obtainable, and are in particularly obtained by a method comprising the steps of: i) mixing and milling *Aspalathus linearis* material in milk or a milk protein-containing liquid medium, ii) optionally separating insoluble material to obtain an aqueous suspension iii) optionally pasteurising the resulting suspension iv) optionally add synthetic or natural bioactive components during the processing v) and further optionally drying the suspension to obtain a powder.

This process has the advantage of being natural and cost effective enabling improved delivery of multi-nutrients in the form of a combination of stabilized water- and fat-soluble compounds in their natural compositions, free of organic solvent residues.

If an extract of *Aspalathus linearis* is used in the present invention it is preferred if at least 50wt.-%, preferably at least 70 wt.-% more preferably at least 95 wt.-% of the extract are water soluble. This has the advantage that the extract can easily be admixed with other water based foodstuffs before consumption.

One typical example of an extract of *Aspalathus linearis* that can be used in the framework of the present invention is rooibos tea.

Suitable extracts of *Aspalathus linearis* for the purpose of the present invention are also extracts that are commercially available, such as for example Rooibos from Afriplex or Rooibos from Rooibos Ltd.

The product comprising a composition comprising *Aspalathus linearis* or an extract thereof according to the present invention may be for example a food product, a drink, a food supplement, a nutraceutical, a cosmetic product, a pet food product or a medicament.

The product may also further comprise a protein source, a carbohydrate source, a lipid source, a mineral source and/or a vitamin source. The presence of proteins, carbohydrates, lipids, minerals and/or vitamins may have several advantages. These compounds generally contribute to the taste and mouthfeel of the final product. They also provide the body with nutrients that it may need urgently when it is affected by inflammatory disorders. They also allow formulating the product of the present invention as a complete nutritional formula, so that no additional nutrition is needed. This might be in particularly helpful for consumers that do not ingest sufficient amounts of food or that have trouble swallowing and that hence wish to ingest only small amounts of food.

Consequently, the products used in the present invention are preferably intended for enteral or for topical application. Also a parenteral administration is possible. While the products are primarily intended to be used by humans, they may also be applied to animals, in particular companion animals, pets or livestock.

Particularly preferred products are products selected from the group consisting of teas, iced teas, a foamed beverage, a confectionery product, a culinary product, a nutritional complete formula, a dairy product, a chilled or shelf stable beverage, a product for lactating mothers, a liquid drink, a soup, a dietary supplement, a meal replacement, a nutritional bar, a milk or a fermented milk product, a yoghurt, a milk based powder, an enteral nutrition product, an infant formula, an infant nutritional product, a puree, a cereal product or a fermented cereal based product, an ice-cream, candy, sweets, biscuits, cakes, a chocolate, a cappuccino, a coffee, a culinary product such as mayonnaise, tomato puree or salad dressings, a pet food, a pet beverage, a tablet, a capsule, a pill, a solution, a suspension, a syrup, a powder, a gel, a cream, a dried oral supplement, a wet oral supplement, a body wash, a tooth paste, a shampoo, a mouth wash, a lipstick, or a personal hygiene product.

The amounts of *Aspalathus linearis* or an extract thereof in the product will depend on several factors, such as the nature of the extract, the condition of the plant, the age, condition and size of the person or animal to be treated, the frequency, the product will be applied and/or the specific kind of inflammatory disorder or condition to be treated or prevented.

The present inventors have found that the effectiveness of *Aspalathus linearis* or an extract thereof according to the present invention is generally dose dependant and follows a dose response curve. If generally mild inflammatory disorders or conditions are to be prevented and the product will be used frequently, very small amounts of *Aspalathus linearis* or an extract thereof will be sufficient to achieve the desired effect. If a severe inflammatory disorder is to be treated, larger amounts of *Aspalathus linearis* or an extract thereof will be more appropriate, although also small amounts will produce an effect.

Generally, it is preferred if the product contains *Aspalathus linearis* or an extract thereof in an amount in the range of about 0,1 g/l to 10 g/l, preferably in the range of 0,5 g/l to 3 g/l product. If the total amount of product cannot be measured in litres it is preferred if the product contains *Aspalathus linearis* or an extract thereof in an amount in the range of about 0,1 g/kg to 10 g/kg, preferably in the range of 0,5 g/kg to 3 g/kg product.

Preferably the product contains *Aspalathus linearis* or an extract thereof in a daily dose of 0,01 g-100g, preferably 0,25 g-10g.

The product may also comprises at least one kind of food grade micro-organism, in particular probiotics.

"Food grade" are all substances that are approved for human or animal consumption.

"Probiotic" means microbial cell preparations or components of microbial cells with a beneficial effect on the health or well-being of the host. (Salminen S, Ouwehand A. Benno Y. et al "Probiotics: how should they be defined" Trends Food Sci. Technol. 1999:10 107-10).

For the purpose of the present invention, the probiotics may be selected from the group consisting of group consisting of Bifidobacterium, Lactobacillus, Streptococcus and Saccharomyces or mixtures thereof, in particular selected from the group consisting of Bifidobacterium longum, Bifidobacterium lactis, Lactobacillus acidophilus, Lactobacillus rhamnosus, Lactobacillus johnsonii, Lactobacillus plantarum, Lactobacillus salivarius, Streptococcus faecium, Saccharomyces boulardii and Lactobacillus reuteri or mixtures thereof, preferably selected from the group consisting of Lactobacillus johnsonii NCC 533 (CNCM I-1225), Bifidobacterium longum NCC 490 (CNCM I-2170), Bifidobacterium longum NCC 2705 (CNCM I-2618) , Bifidobacterium lactis Bb12, Bifidobacterium lactis NCC2818 (CNCM I-3446), Lactobacillus paracasei NCC 2461 (CNCM I-2116), Lactobacillus rhamnosus GG, Lactobacillus rhamnosus NCC4007 (CGMCC 1.3724) Enterococcus faecium SF 68 (NCIMB 10415), and mixtures thereof.

The presence of probiotics in a composition comprising *Aspalathus linearis* or an extract thereof may have several important effects. Generally the benefits of probiotics are known to be often strain specific and include the treatment or prevention allergies, atopic eczema, diarrhoea, Irritable Bowel Syndrome, metabolic disorders and/or obesity, all of which may be in part related to inflammatory disorders. Consequently, the combination of probiotics with *Aspalathus linearis* or an extract thereof can be expected to have a further positive effect on the treatment and prevention of inflammatory disorders.

The product may also contain at least one prebiotic.

"Prebiotic" means food substances that promote the growth of probiotics in the intestines. They are not broken down in the stomach and/or upper intestine or absorbed in the GI tract of the person ingesting them, but they are fermented by the gastrointestinal microflora and/or by probiotics. Prebiotics are for example defined by Glenn R. Gibson and Marcel B. Roberfroid, Dietary Modulation of the Human Colonic Microbiota: Introducing the Concept of Prebiotics, J. Nutr. 1995 125: 1401-1412.

Prebiotics are known to produce a synergistic effect with probiotics, thus the combination of probiotics, prebiotics and *Aspalathus linearis* or an extract thereof will be very effective in treating or preventing inflammatory disorders. The prebiotics that may be used in accordance with the present inventions are not particularly limited and include all food substances that promote the growth of probiotics in the intestines. Preferably, they may be selected from the group consisting of oligosaccharides, optionally containing fructose, galactose, mannose; dietary fibers, in particular soluble fibers, soy fibers; inulin; or mixtures thereof. Preferred prebiotics are fructo-oligosaccharides (FOS), galacto-oligosaccharides (IOS), isomalto-oligosaccharides, xylo-oligosaccharides, oligosaccharides of soy, glycosylsucrose (GS), lactosucrose (LS), lactulose (LA), palatinose-oligosaccharides (PAO), malto-oligosaccharides (MOS), gums and/or hydrolysates thereof, pectins and/or hydrolysates thereof.

Finally, the product prepared for the use of the present invention may also contain at least one phytonutrient.

The term "phytonutrient" describes those plant compounds which have health-protecting qualities. For example antioxidant, immune boosting or other health promoting properties of active compounds in plants are typical effects of phytonutrients. Phytonutrients include but are not limited to terpenes, carotenoids, limonoids, polyphenols, and phytosterols.

The carotenoids may be selected from the group consisting of carotenes and xanthophylls such as lycopene, carotene, phytofluene, phytoene, canthaxanthin, beta-cryptoxanthin, capsanthin, lutein, zeaxanthin, or those in the form of fatty acid esters, or mixtures thereof.

The polyphenols are preferably selected from the group consisting of flavones such as apigenin, luteolin or diosmetin, flavonols such as quercetin, myricetin, kaempferol, flavanones such as naringenin or hesperetin, flavanols such as catechin, epicatechin, epigallocatechin, anthocyanidins such as pelargonidin, malvidin, or isoflavones such as genistein, daidzein, or phenolic acids such as chlorogenic, caffeic, or ferulic acids or mixtures thereof.

Preferred phytonutrients for the present invention are beta-carotene, lutein, and/or catechin. The phytonutrients may be also provided in the form of a plant or plant extract.

It is clear to those of skill in the art that they can freely combine all features described herein without departing from the scope of the present invention as disclosed. In particular, features described for the use of the present invention may be applied to the composition comprising *Aspalathus linearis* or an extract thereof or the product comprising the composition comprising *Aspalathus linearis* or an extract thereof and vice versa.

Further advantages and features of the present invention are apparent from the following examples and figures.

Figure 1 shows the ICAM1 and IL-8 mRNA expression in HT-29 clone 34 cells stimulated by TNFα (10ng/ml) and treated with different dosages of Rooibos-4 from Afriplex (left panel) and Rooibos-10 from Rooibos Ltd. (right panel).

Figure 2 shows that water extracts from rooibos tea (Rooibos-4 from Afriplex, in black, and Rooibos-10 from Rooibos Ltd., in horizontal hatched bar) at a concentration of 50 and 500µg/ml significantly (p<0.05) decreased the amount of GAG released in the supernatants in 4 independent experiments. The effects of the 2 extracts at 500µg/ml were similar to the effects of the positive control (in grey).

Figure 3 shows the effect of fermented rooibos on the induction of Heme-oxygenase 1. A dose-dependent induction of HO-1 was observed after treatment of primary hepatocytes with fermented rooibos.

### Example 1:

To demonstrate the beneficial effect of Rooibos extracts against inflammatory disorders an *in vitro* cell based assay, namely the human intestinal cell line HT-29 NFκB-reporter gene assay, was used.

Briefly, stimulation of HT-29 cells with TNFα (proinflammatory stimulus at 10 ng/ml) induced the expression of ICAM1 and IL-8 mRNA. To assess the activity of Rooibos, HT-29 cells were pre-incubated with different concentrations of this compound. After 2 h of treatment, TNFα (10 ng/ml) was added into the cell culture medium and HT-29 cells were further incubated with for 3h and 5h. After 5h, the viability of the cells was assessed and an mRNA extraction was performed in order to quantify ICAM1 and IL-8 expression by real time PCR.

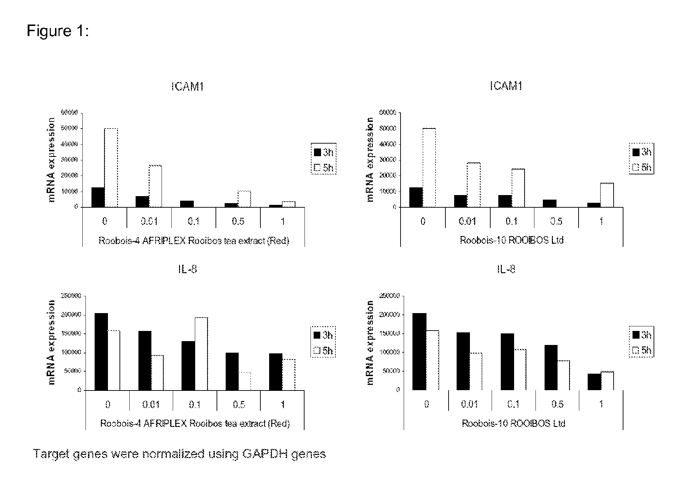

Figure 1 shows the results of this study and demonstrates the effect of *Aspalathus linearis* or an extract thereof against inflammatory disorders.

### Example 2:

Two extracts from rooibos tea were shown to decrease cytokine-induced cartilage destruction *in vitro* as assessed by the amount of glycosaminoglycans released in culture medium, demonstrating the effectiveness of *Aspalathus linearis* or an extract thereof against arthritis.

This experiment was performed as follows: Articular cartilage explants are dissected out of the metacarpophalangeal joint of old cows (8-10 years). The skin is removed from the feet. The articulation is opened transversally. Intra articular ligaments are transected. Full thickness slices of cartilage are dissected out and put in a Petri dish containing DMEM supplemented with 20% FBS (fetal bovine serum albumin) and antibiotics (penicillin, streptomycin and gentamycin). Under the hood, disks of cartilage obtained by using a biopsy punch (6 mm in diameter) are distributed between the wells of a 96-wells plate containing 200µl of medium (DMEM + 20% FBS, 1% penicillin/streptomycin, 0.1% gentamycin) per well. Finally, plates are put into an incubator (37°C / 5% CO₂).

Five days after their harvest, the explants are divided into several different treatment conditions:
1- positive inhibitory control: d15-PGJ2 (a peroxisome proliferator activated receptor γ agonist) and hymenialdisine are co-added, to the medium, with IL1β, to counteract the catabolic effect of this cytokine. (Sabatini M, et al. (2002) Osteoarthritis Cartilage 10(9): 673-679; Scher JU, et al. (2005) Clin Immunol 114(2): 100-109; Boyault S, et al. (2001) Febs Letters 501(1): 24-30)
2, 3, 4- tested compounds: these conditions test the effects of three concentrations of two different rooibos extracts in presence of IL1β.
The explants undergo these treatments for 72 hours in the incubator (37°C, 5%CO₂). The following protocol is based on the disclosure of Campbell et al. (1984) Arch Biochem Biophys 234(1): 275-289 and Hascall VC, et al. (1983) ArchBiochem Biophys 224(1): 206-223 and was carried out 48 hours after the harvest of the explants.
Sulphur 35 (35S) was added to the medium to be incorporated into newly synthesized polysulfated glycosaminoglycans within the extracellular matrix of the explants. After 72 hours, several washes were performed to eliminate the non-incorporated radioactivity. The explants were then treated for 72hours, after which the supernatants were collected and the amount of radioactivity released in the medium was measured with a beta counter, as a marker of glycosaminoglycan (GAG) catabolism. The radioactivity present in the explants was also measured and used to normalize the amount of radioactivity released in the media. The radioactivity was expressed in DPM (degradations per minute) and the GAG release was assessed by the following formula:

GAG release = radioactivity in the supernatants / (radioactivity in the supernatants + radioactivity in the explants)

After being loaded with radioactivity, cartilage explants were treated for 72 hours with DMEM only or with 50ng/ml of IL1β only (horizontal bar) or with IL1β and PGJ2 and hymenialdisine (grey bar), a positive control of inhibition, or with IL1β and rooibos extracts (black and horizontal hatched bars). The dose range of the rooibos extracts was: 500 - 50 -5 µg/ml. Data are expressed as the percentage of radioactivity release (mean of 6 culture wells ± standard deviation), with percentage of radioactivity release = supernatant radioactivity / (supernatant radioactivity + explants radioactivity). The percentage of radioactivity released is used as a surrogate marker for GAG release. Three independent experiments are represented.

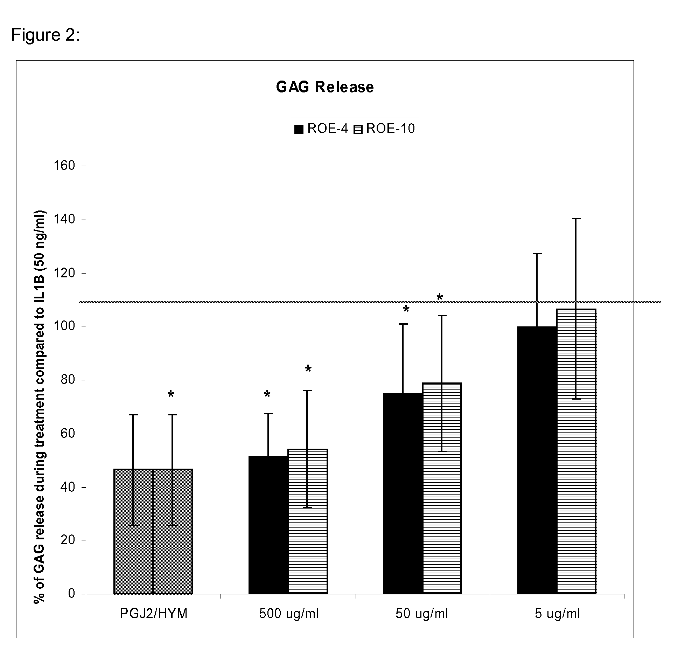

The stimulation by 50 ng/ml of interleukin 1 beta (IL1β) significantly increased the basal GAG release in the media. Compared to IL1β alone (horizontal black line), 2 water extracts from rooibos tea (Rooibos-4 from Afriplex, in black, and Rooibos 10 from Rooibos Ltd, in horizontal hatched bars) at a concentration of 50 and 500µg/ml significantly (p<0.05) decreased the amount of GAG released in the supernatants in 4 independent experiments (see average results from these experiments in Figure 2). The effects of the 2 extracts at 500µg/ml were similar to the effects of the positive control (in grey).

### Example 3:

Heme oxygenase (HO) is the rate limiting enzyme in the breakdown of heme into carbon monoxide (CO), iron and bilirubin. Previous studies have shown that induction of HO-1 is associated with a protective response due to (1) the removal of free heme, which is shown to be toxic, (2) the generation of bilirubin a potent antioxidant, antimutagen and anti-complement agent playing a cytoprotective role against free radical damage as well as (3) the generation of CO a critical intracellular signaling molecule, and a potent vasodilator.

It has been recently demonstrated that a pre-induction of HO activity ameliorates inflammation. Moreover, induction of HO-1 in response to different inflammatory mediators could contribute in part to the resolution of inflammation (Lee,T.S., et al., J. Biol. Chem. 278, 19325-19330; Kapturczak,M.H., et al., 2004, Am. J Pathol. 165, 1045-1053; Naito,Y., et al., 2004, Aliment. Pharmacol. Ther. 20 Suppl 1, 177-184).

### Cell culture

### Primary rat hepatocytes

Primary isolated hepatocytes were obtained by perfusion of the liver in adult male Sprague-Dawley (230 +/- 20g, fed *ad libitum* with Nafag diet) with a collagenase solution. Cell viability, estimated by Trypan blue exclusion test, was found to range between 90 and 95%. Cells were seeded at a density of 10⁶ cells/ ml on 60-mm plastic tissue culture dishes in 3 ml William's E medium supplemented with 2 mM L-glutamine, 10 mM HEPES, pH 7.4, 1 % ITS+ 100 U/ ml penicillin/ streptomycin, 100 nM dexamethasone and fetal bovine serum 5% (Hi-clone). Hepatocytes were allowed to attach for 2 h and then washed with Earle's balanced salt solution to remove debris and unattached cells. Fresh serum-free medium containing 250 nM dexamethasone was added followed by application of an overlay of matrigel (233 g/ml). Fresh matrigel was added to the cultures every other day following medium change. To study the effects of fermented *Aspalathus linearis* (rooibos) extracts on specific markers, the extract was added to culture media containing ITS+ 0.2% 24 h after cells seeding over periods of 24h.

### Real-time polymerase chain reaction (PCR)

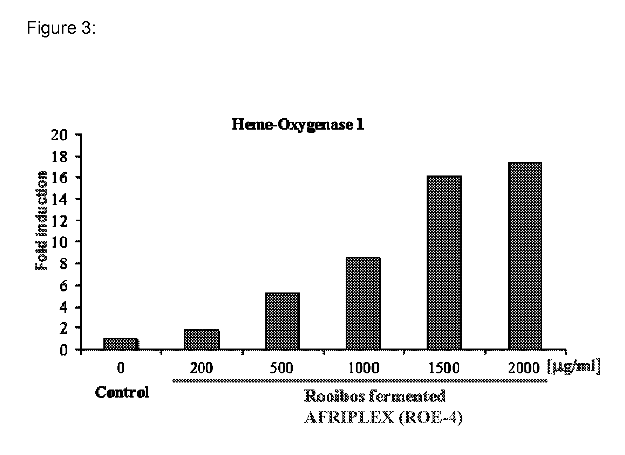

RNA from primary hepatocytes treated and non-treated with extracts was extracted using the Nucleospin system following the suppliers protocol (Nucleospin Inc.). RNA was converted to cDNA using the two-step TaqMan Gold RT-PCR system (Applied Biosystem, Foster City, CA) containing the TaqMan Reverse Transcription Reagents and the TaqMan PCR Core kit for relative quantification experiments. Quantification of amplified PCR products was performed using ABI Prism®7000 Sequence Detection System software Version 1.1, normalized to the rat 18S gene as internal control. PCR probe for heme-oxygenase 1 was obtained from the Assays on Demand (AoD) Service of Applied Biosystems. Results are shown in figure 3.

## Claims

1. Use of a composition comprising *Aspalathus linearis* or an extract thereof for the preparation of a product to treat and/or prevent inflammatory disorders.

2. Use in accordance with claim 1, wherein the product is to treat and/or prevent inflammatory disorders of the gut, in particular inflammatory bowel syndrome; and/or of the cartilage; in particular osteoarthritis and/or rheumatoid arthritis; and/or the symptoms thereof.

3. Use in accordance with one of the preceding claims, to modulate the ratio of cartilage anabolism and cartilage catabolism, in particular to increase the ratio of cartilage anabolism and cartilage catabolism, and/or to inhibit cartilage catabolism.

4. Use in accordance with one of the preceding claims, wherein the extract is obtainable by extraction with a solvent from *Aspalathus linearis* plant material, in particular by a water extraction or an alcohol/water extraction, for example by a ethanol/water extraction.

5. Use in accordance with one of the preceding claims, wherein at least 50wt.-%, preferably at least 70 wt.-% more preferably at least 95 wt.-% of the extract are water soluble.

6. Use in accordance with one of the preceding claims, wherein the extract is a commercially available extract, preferably Rooibos from Afriplex or Rooibos from Rooibos Ltd.

7. Use in accordance with one of the preceding claims, wherein the product is a food product, a drink, a food supplement, a nutraceutical, a cosmetic product, a pet food product or a medicament.

8. Use in accordance one of the preceding claims, wherein the product is selected from the group consisting of teas, iced teas, a foamed beverage, a confectionery product, a culinary product, a nutritional complete formula, a dairy product, a chilled or shelf stable beverage, a product for lactating mothers, a liquid drink, a soup, a dietary supplement, a meal replacement, a nutritional bar, a milk or a fermented milk product, a yoghurt, a milk based powder, an enteral nutrition product, an infant formula, an infant nutritional product, a puree, a cereal product or a fermented cereal based product, an ice-cream, candy, sweets, biscuits, cakes, a chocolate, a cappuccino, a coffee, a culinary product such as mayonnaise, tomato puree or salad dressings, a pet food, a pet beverage, a tablet, a capsule, a pill, a solution, a suspension, a syrup, a powder, a gel, a cream, a dried oral supplement, a wet oral supplement, a body wash, a tooth paste, a shampoo, a mouth wash, a lipstick, or a personal hygiene product.

9. Use in accordance with one of the preceding claims, wherein the product contains *Aspalathus linearis* or an extract thereof in an amount in the range of about 0,1 g/l to 10 g/l, preferably in the range of 0,5 g/l to 3 g/l, and/or wherein the product contains *Aspalathus linearis* or an extract thereof in a daily dose of 0,01 g-100g, preferably 0,25 g-10g.

10. Use in accordance with one of the preceding claims, wherein the product further comprises a protein source, a carbohydrate source, a lipid source, a mineral source and/or a vitamin source and is for oral, enteral and/or parenteral application.

11. Use in accordance with one of the preceding claims, wherein the product comprises at least one kind of food grade micro-organisms, in particular probiotics.

12. Use in accordance with claim 10, **characterized in that** the probiotics are selected from the group consisting of group consisting of Bifidobacterium, Lactobacillus, Streptococcus and Saccharomyces or mixtures thereof, in particular selected from the group consisting of Bifidobacterium longum, Bifidobacterium lactis, Lactobacillus acidophilus, Lactobacillus rhamnosus, Lactobacillus johnsonii, Lactobacillus plantarum, Lactobacillus salivarius, Streptococcus faecium, Saccharomyces boulardii and Lactobacillus reuteri or mixtures thereof, preferably selected from the group consisting of Lactobacillus johnsonii NCC 533 (CNCM I-1225), Bifidobacterium longum NCC 490 (CNCM I-2170), Bifidobacterium longum NCC 2705 (CNCM I-2618) , Bifidobacterium lactis Bb12, Bifidobacterium lactis NCC2818 (CNCM I-3446), Lactobacillus paracasei NCC 2461 (CNCM I-2116), Lactobacillus rhamnosus GG, Lactobacillus rhamnosus NCC4007 (CGMCC 1.3724) Enterococcus faecium SF 68 (NCIMB 10415), and mixtures thereof.

13. Use in accordance with one of the preceding claims, wherein the product further contains at least one prebiotic.

14. Use in accordance with claim 12 **characterized in that** the prebiotic is selected from the group consisting of oligosaccharides, optionally containing fructose, galactose, mannose; dietary fibers, in particular soluble fibers, soy fibers; inulin; or mixtures thereof.

15. Use in accordance with one of the preceding claims, wherein the product further contains at least one phytonutrient, preferably selected from the group consisting of lutein and catechin.
